# EUROPEAN PATENT SPECIFICATION

(11) **EP 4 496 532 B1**
(45) Date of publication and mention of the grant of the patent: **28.10.2026**
(21) Application number: 23716671.5
(22) Date of filing: 21.03.2023
(51) Int. Cl.: A61F 2/16, B82Y 20/00

(54) **INTRAOCULAR LENSES WITH NANOSTRUCTURES AND METHODS OF FABRICATING THE SAME**
INTRAOKULARLINSEN MIT NANOSTRUKTUREN UND HERSTELLUNGSVERFAHREN DAFÜR
LENTILLES INTRAOCULAIRES AVEC NANOSTRUCTURES ET LEURS PROCÉDÉS DE FABRICATION

(30) Priority: 22.03.2022 US 202263269719 P; 29.04.2022 US 202263363828 P; 17.05.2022 US 202263364813 P
(43) Date of publication of application: 29.01.2025
(73) Proprietor: Alcon Inc., 1701 Fribourg (CH)
(72) Inventor: ZHANG, Hao, Fort Worth, Texas 76134 (US); HONG, Xin, Fort Worth, Texas 76134 (US); PALIWAL, Sumit, Fort Worth, Texas 76134 (US)
(74) Representative: Murgitroyd & Company
(86) International application number: PCT/IB2023/052774
(87) International publication number: WO 2023/180934

(56) References cited:
- EP-B1- 2 166 986
- EP-B1- 2 693 982
- WO-A1-2020/206343
- DE-C2- 3 428 895

## Description

### BACKGROUND

The human eye in its simplest terms functions to provide vision by transmitting light through a clear outer portion called the cornea, and focusing the image by way of a lens onto a retina. The quality of the focused image depends on many factors including the size and shape of the eye, and the transparency of the cornea and lens. When age or disease causes the lens to become less transparent, vision deteriorates because of the diminished light which can be transmitted to the retina. This deficiency in the lens of the eye is medically known as a cataract. An accepted treatment for this condition is surgical removal of the lens and replacement of the lens function by an intraocular lenses (IOLs).

Although existing IOLs as well as methods and systems for manufacturing thereof may be acceptable, they also have certain shortcomings. Accordingly, there is a need for improvements to IOL designs and associated manufacturing techniques for complex optical designs.
Reference is made to the documents EP 2 693 982 B1, DE 34 28 895 C2, EP 2 166 986 B1, and WO 2020/206343 A1 which have been cited as exemplary of the state of the art. Particularly, EP 2 693 982 relates to an IOL provided with nanostructures on its anterior and posterior surfaces.

### SUMMARY

The scope of the invention is in accordance with the appended claims.

Aspects of the present disclosure provide an intraocular lens (IOL) including a lens body having a monolithic anterior lens element having an anterior nanostructure assembly formed thereon and a monolithic posterior lens element having a posterior nanostructure assembly formed thereon, and one or more haptics coupled to the lens body.

Aspects of the present disclosure also provide a method for fabricating an intraocular lens (IOL). The method includes fabricating a monolithic anterior lens element and a monolithic posterior lens element, bonding the monolithic anterior lens element and the monolithic posterior lens element to form a cavity therebetweeen, and filling the cavity with an optical fluid. The monolithic anterior lens element has an anterior nanostructure assembly formed thereon, and the monolithic posterior lens element has a posterior nanostructure assembly formed thereon.
Aspects of the present disclosure further provide a method for configuring an intraocular lens (IOL). The method includes computing configurations of an anterior nanostructure assembly formed on an anterior lens element of an IOL and a posterior nanostructure assembly formed on a posterior lens element of the IOL, based on a lens base poser and a desired value of refractive index of the IOL, and forming the IOL or causing the IOL to be formed based on the computed configurations of the anterior nanostructure assembly and the posterior nanostructure assembly.

### BRIEF DESCRIPTION OF THE DRAWINGS

So that the manner in which the above recited features of the present disclosure can be understood in detail, a more particular description of the disclosure, briefly summarized above, may be had by reference to embodiments, some of which are illustrated in the appended drawings. It is noted, however, that the appended drawings illustrate only some aspects of this disclosure and the disclosure may admit to other equally effective embodiments.
Figure 1A depicts a top view of an intraocular lens (IOL), according to certain embodiments.
Figure 1B depicts a side view of a portion of the IOL of Figure 1A, according to certain embodiments.
Figure 1C depicts an enlarged view of a portion of the IOL of Figure 1A, according to certain embodiments.
Figure 2 depicts example operations for fabricating an IOL with nanostructures, according to certain embodiments.
Figure 3 depicts example operations for forming a lens element, according to certain embodiments.
Figures 4A, 4B, and 4C illustrate various aspects of a lens element corresponding to the different stages of the operations of Figure 3, according to certain embodiments.
Figure 5 depicts an example system for designing, configuring, and/or forming an IOL, according to certain embodiments.
Figure 6 depicts example operations for forming an IOL, according to certain embodiments.

To facilitate understanding, identical reference numerals have been used, where possible, to designate identical elements that are common to the figures. It is contemplated that elements and features of one embodiment may be beneficially incorporated in other embodiments without further recitation.

### DETAILED DESCRIPTION

The present disclosure provides methods and systems for fabricating an intraocular lens (IOL) having nanostructured patterns embossed onto on its external surfaces of the IOL. In certain embodiments, the methods and the system include producing an anterior lens element and a posterior lens element, each having nanostructured patterns, bonding the anterior and posterior lens elements to form a cavity therebetween, and filling the cavity with an optical fluid.

### An IOL with Nanostructures

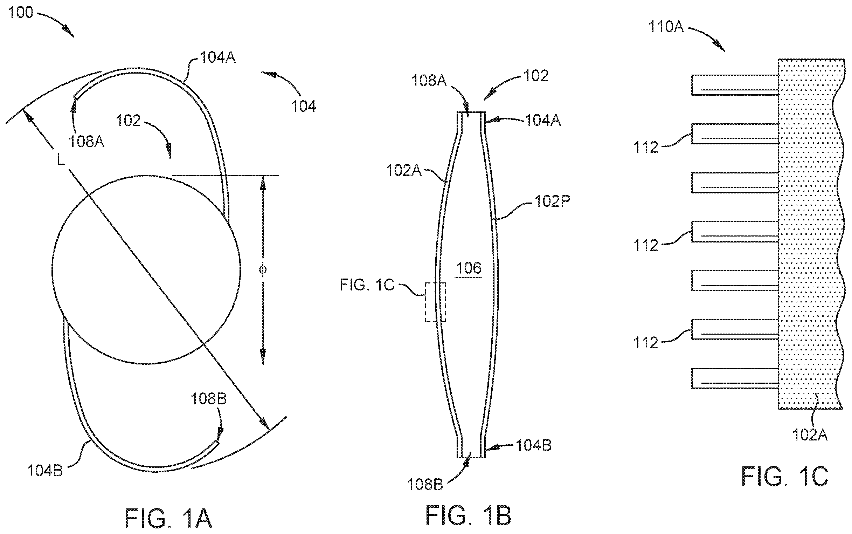

Figure 1A illustrates a top view of an intraocular lens (IOL) 100, according to certain embodiments. Figure 1B illustrates a side view of a portion of the IOL 100. FIG. 1C illustrates an enlarged side view of a portion of the IOL 100. The IOL 100 includes a lens body 102 and a haptic portion 104 that is coupled to a peripheral, non-optic portion of the lens body 102.

The lens body 102 includes an anterior lens element 102A and a posterior lens element 102P. The lens elements 102A and 102P are bonded together to form a cavity 106. The cavity 106 is filled with an optical fluid. The optical fluid may be an incompressible or substantially incompressible fluid exhibiting a refractive index that is different from the lens elements 102A and 102P. The optical fluid may be a refractive index-matched silicone oil of ophthalmic grade, such as the optical fluid available from Entegris, Inc., Billerica, Massachusetts. The anterior lens element 102A and the posterior lens element 102P may be fabricated of a transparent, flexible, biocompatible polymer, such as flexible polymer, including poly (dimethylsiloxane) (PDMS). The lens body 102 has a diameter *ϕ* of between about 4.5 mm and about 7.5 mm, for example, about 6.0 mm.

The haptic portion 104 includes hollow radially-extending struts (also referred to as "haptics") 104A and 104B that are coupled (e.g., glued or welded) to the peripheral portion of the lens body 102 or molded along with a portion of the lens body 102, and thus extend outwardly from the lens body 102 to engage the perimeter wall of the capsular sac of the eye to maintain the lens body 102 in a desired position in the eye. The haptics 104A and 104B each have an internal volume 108A, 108B, which is in fluid communication with the cavity 106 of the lens body 102. The haptics 104A and 104B may be fabricated of biocompatible material, such as modified poly (methyl methacrylate) (PMMA), modified PMMA hydrogels, hydroxy-ethyl methacrylate (HEMA), PVA hydrogels, other silicone polymeric materials, hydrophobic acrylic polymeric materials, for example, AcrySof^{®} and Clareon^{®}, available from Alcon, Inc., Fort Worth, Texas. The haptics 104A and 104B typically have radial-outward ends that define arcuate terminal portions. The terminal portions of the haptics 104A and 104B may be separated by a length L of between about 6 mm and about 22 mm, for example, about 13 mm. The haptics 104A and 104B have a particular length so that the terminal portions create a slight engagement pressure when in contact with the equatorial region of the capsular sac after being implanted. While Figure 1A illustrates one example configuration of the haptics 104A and 104B, any plate haptics or other types of haptics can be used.

It is noted that the shape and curvatures of the lens body 102 are shown for illustrative purposes only and that other shapes and curvatures are also within the scope of this disclosure. For example, the lens body 102 shown in Figure 1B has a bi-convex shape. In other examples, the lens body 102 may have a plano-convex shape, a convexo-concave shape, or a plano-concave shape. In certain embodiments, as shown in Figure 1B, the IOL 100 is a mono-focal IOL (with a single focal point) having no annular echelettes formed on the anterior lens element 102A or the posterior lens element 102P. In some other embodiments, the IOL 100 is a multi-focal IOL (with multiple focal points, e.g., bi-focal and tri-focal) having annular echelettes on the anterior lens element 102A and/or the posterior lens element 102P (not shown). In some other embodiments, the IOL 100 is an extended depth of focus (EDOF) IOL (with elongated focus) having annular echelettes on the posterior lens element 102P (not shown).

In the embodiments described herein, the anterior lens element 102A includes an anterior nanostructure assembly 110A formed thereon, and the posterior lens element 102P includes a posterior nanostructure assembly 110P formed thereon (not shown). The anterior nanostructure assembly 110A and the anterior lens element 102A are formed as a monolithic single piece of the same material, such as flexible polymer, including PDMS. The posterior nanostructure assembly 110P and the posterior lens element 102P are also formed as a monolithic single piece of the material, such as flexible polymer, including PDMS. In certain embodiments, the lens body 102 includes only one of the anterior nanostructure assembly 110A or the posterior nanostructure assembly 110P. The lens element 102A, or 102P that does not have a nanostructure assembly formed thereon may include a diffractive structure to adjust a refractive index and/or reflectivity of the lens.

As shown in Figure 1C, the anterior nanostructure assembly 110A includes protrusions 112. The posterior nanostructure assembly 110P includes similar protrusions 112. Shapes, sizes, and density (e.g., spacing between adjacent protrusions 112) of the protrusions 112 are designed for the lens body 102 to provide a desired refractive index. For example, the protrusions 112 may have heights H of between about 30 nm and about 200 nm, widths of between about 30 nm and about 300 nm, and spacings between adjacent protrusions 112 of between about 30 nm and about 300 nm. The nanostructure assemblies 110A, 110P may further reduce reflectivity by between about 10 % and about 90 %, as compared to a lens body having no nanostructure assemblies on its anterior outer surface or posterior outer surface. In certain embodiments, as shown in Figure 1B, the nanostructure assemblies 110A, 110P entirely cover the anterior lens element 102A and the posterior lens element 102P, respectively. However, in some other embodiments, the nanostructure assemblies 110A, 110P only partially cover the anterior lens element 102A and the posterior lens element 102P, respectively.

### Fabrication of an IOL with Nanostructures

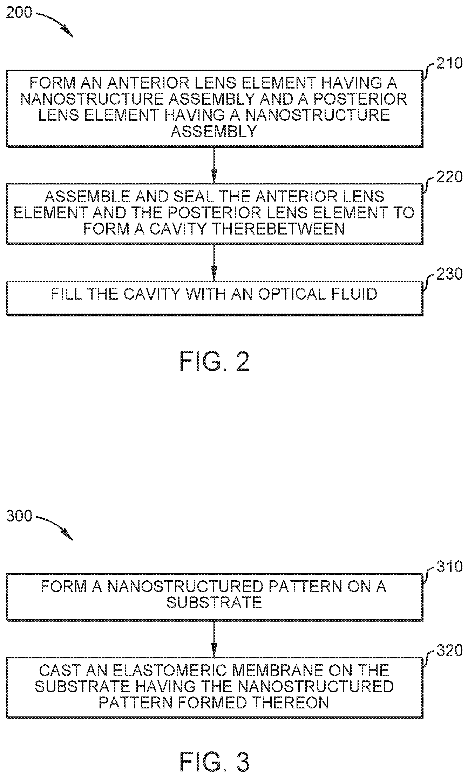

Figure 2 depicts example operations 200 for forming an IOL with nanostructures, according to certain embodiments.

At step 210, an anterior lens element (e.g., anterior lens element 102A) having a nanostructure assembly (e.g., nanostructure assembly 110A (shown in Figure 1C)) and a posterior lens element (e.g., posterior lens element 102P) having a nanostructure assembly (e.g., nanostructure assembly 110P) are formed, as described below in relation to operations 300.

At step 220, the anterior lens element and the posterior lens element are assembled and sealed to form a cavity (e.g., cavity 106 (shown in Figure 1B)). The anterior lens element and the posterior lens element may be bonded together to make a seal in a peripheral non-optic portion of the lens body (e.g., lens body 102), by chemical bonding, thermal bonding, UV bonding or other appropriate types of bonding, with the cavity therebetween.

At step 230, the cavity is filled with an optical fluid, like silicone oil.

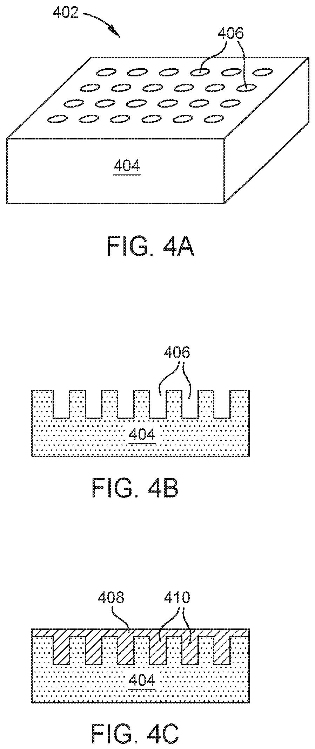

Figure 3 depicts example operations 300 for forming each of the lens elements 102A, 102P (i.e., step 210). Figures 4A, 4B, and 4C illustrate various aspects of a lens element corresponding to the different stages of the operations 300. As such, Figures 3, 4A, 4B, and 4C are described together.

At step 310, a nanostructured pattern (e.g., nanostructured pattern 402) is formed on a substrate (e.g., substrate 404) by standard micro/nano fabrication methods, as shown in an isometric view in Figure 4A and a cross-sectional view in Figure 4B. For example, first, a photolithography process is performed to spin on a photoresist on the substrate, and selected areas of the photoresist are exposed to light and developed. Second, the pattern of the photoresist is transferred to the substrate by reactive ion etching. Then, the remaining photoresist is removed by plasma over-etching. The nanostructured pattern may extend over an area of about 7 mm and about 7 mm on the substrate, and include an array of trenches (e.g., array of trenches 406) carved in the substrate with heights of between about 30 nm and about 200 nm, widths of between about 30 nm and about 300 nm, and spacings between adjacent trenches of between about 30 nm and about 300 nm.

The term "substrate" as used herein refers to a layer of material that serves as a basis for subsequent processing operations and includes a surface to be cleaned. For example, the substrate may include glass, or one or more conductive metals, such as nickel, titanium, platinum, molybdenum, rhenium, osmium, chromium, iron, aluminum, copper, tungsten, or combinations thereof. The substrate can also include one or more materials comprising silicon, including materials associated with group IV or group III-V including compounds, such as Si, polysilicon, amorphous silicon, silicon nitride, silicon oxynitride, silicon oxide, Ge, SiGe, GaAs, InP, InAs, GaAs, GaP, InGaAs, InGaAsP, GaSb, InSb and the like, or combinations thereof. Furthermore, the substrate can also include dielectric materials such as silicon dioxide, organosilicates, and carbon doped silicon oxides. Further, the substrate can include any other materials such as metal nitrides, metal oxides and metal alloys, depending on the application.

Moreover, the substrate is not limited to any particular size or shape. The substrate can be a round wafer having a 200 mm diameter, a 300 mm diameter, a 450 mm diameter or other diameters. The substrate can also be any polygonal, square, rectangular, curved or otherwise non-circular workpiece, such as a polygonal glass, plastic substrate.

At step 320, an elastomeric membrane (e.g., elastomeric membrane 408) is casted on the substrate having the nanostructured pattern formed thereon, as shown in exemplary Figure 4C. The elastomeric membrane may be formed of a thin PDMS. In the casting process, PDMS in a liquid form may be mixed with a cross-linking agent and poured onto the substrate and heated at an elevated temperature of between about 250 °C and about 350 °C, hardening and cross-linking PDMS, to form the elastomeric membrane. The elastomeric membrane may have a thickness of between about 200 µm and 500 µm. The elastomeric membrane has protrusions (e.g., protrusions 410) that replicate the nanostructured pattern on the substrate. The protrusions may have heights of between about 30 nm and about 200 nm, widths of between about 30 nm and about 300 nm, and spacings between adjacent trenches of between about 30 nm and about 300 nm. After casting, the elastomeric membrane is removed from the substrate. This elastomeric membrane can be used as the anterior lens element having the anterior nanostructure assembly or the posterior lens element having the posterior nanostructure assembly (shown in exemplary Figure 1C).

### System for Designing an IOL

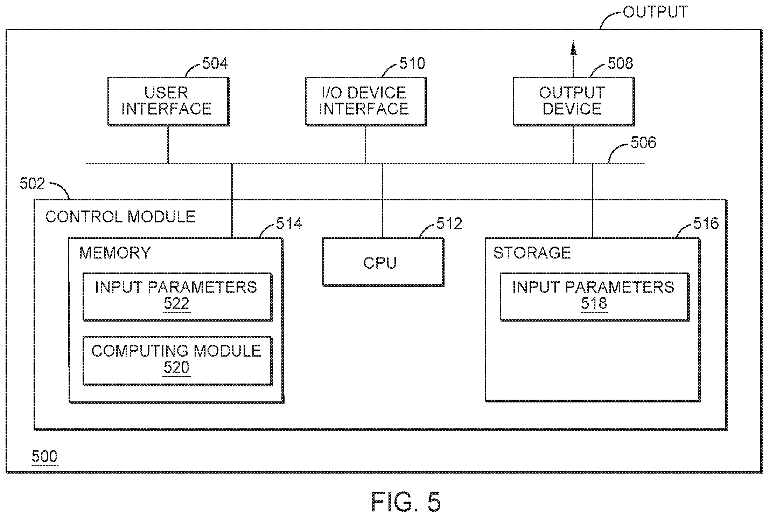

Figure 5 depicts an exemplary system 500 for designing, configuring, and/or forming an IOL 100. As shown, the system 500 includes, without limitation, a control module 502, a user interface display 504, an interconnect 506, an output device 508, and at least one I/O device interface 510, which may allow for the connection of various I/O devices (e.g., keyboards, displays, mouse devices, pen input, etc.) to the system 500.

The control module 502 includes a central processing unit (CPU) 512, a memory 514, and a storage 516. The CPU 512 may retrieve and execute programming instructions stored in the memory 514. Similarly, the CPU 512 may retrieve and store application data residing in the memory 514. The interconnect 506 transmits programming instructions and application data, among CPU 512, the I/O device interface 510, the user interface display 504, the memory 514, the storage 516, output device 508, etc. The CPU 512 can represent a single CPU, multiple CPUs, a single CPU having multiple processing cores, and the like. Additionally, in certain embodiments, the memory 514 represents volatile memory, such as random access memory. Furthermore, in certain embodiments, the storage 516 may be non-volatile memory, such as a disk drive, solid state drive, or a collection of storage devices distributed across multiple storage systems.

As shown, the storage 516 includes input parameters 518. The input parameters 518 include a lens base power and a desired value of refractive index of a lens body. The memory 514 includes a computing module 520 for computing control parameters, such as configuration of the nanostructure assemblies 110A, 110P (e.g., shapes, sizes, and density). In addition, the memory 514 includes input parameters 522.

In certain embodiments, input parameters 522 correspond to input parameters 518 or at least a subset thereof. In such embodiments, during the computation of the control parameters, the input parameters 522 are retrieved from the storage 516 and executed in the memory 514. In such an example, the computing module 520 comprises executable instructions (e.g., including one or more of the formulas described herein) for computing the control parameters, based on the input parameters 522. In certain other embodiments, input parameters 522 correspond to parameters received from a user through user interface display 504. In such embodiments, the computing module 520 comprises executable instructions for computing the control parameters, based on information received from the user interface display 504.

In certain embodiments, the computed control parameters, are output via the output device 508 to a lens manufacturing system that is configured to receive the control parameters and form a lens accordingly. In certain other embodiments, the system 500 itself is representative of at least a part of a lens manufacturing systems. In such embodiments, the control module 502 then causes hardware components (not shown) of system 500 to form the lens according to the control parameters by the operations 200 described above.

### Method for Forming an IOL

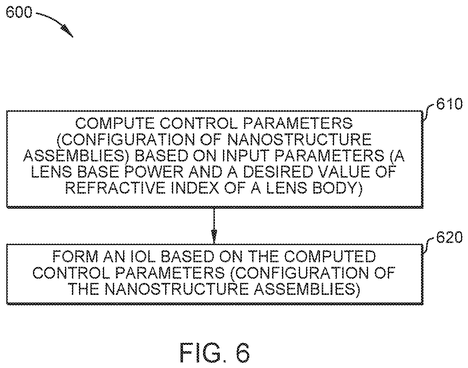

Figure 6 depicts example operations 600 for forming an IOL *(e.g.,* IOL 100). In some embodiments, the step 610 of operations 600 is performed by one system (e.g., the system 500) while step 620 is performed by a lens manufacturing system. In some other embodiments, both steps 610 and 620 are performed by a lens manufacturing system.

At step 610, control parameters, such as configuration of the nanostructure assemblies 110A, 110P (e.g., shapes, sizes, and density) are computed based on input parameters (e.g., a lens base power and a desired value of refractive index of a lens body). The computations performed at step 610 are based on one or more of the embodiments, including the formulas, described herein.

At step 620, an IOL (*e.g.,* IOL 100) based on the computed control parameters, such as configuration of the nanostructure assemblies 110A, 110P (e.g., shapes, sizes, and density) is formed according to the operations 200 described above, using appropriate methods, systems, and devices typically used for manufacturing lenses, as known to one of ordinary skill in the art.

The embodiments described herein provide methods and systems for fabricating an IOL having nanostructured patterns embossed onto the external surfaces of the IOL, by producing an anterior lens element and a posterior lens element, each having nanostructured patterns, bonding the anterior and posterior lens elements to form a cavity therebetween, and filling the cavity with an optical fluid. The methods described herein may offer simplified fabrication processes of IOLs with nanostructured patterns embossed on external surfaces as compared to the conventional fabrication processes. Additionally, the described methods may further eliminate concerns associated with disintegration of implanted IOLs that were formed by separately fabricating nanostructures which are subsequently attached to the IOL. Therefore, the improved manufacturing techniques disclosed herein allow for manufacturing improved IOLs with nanostructures on the external surfaces for reducing customer complaints about glare and halo, as well as issues with reflection possibly associated with the scary eye phenomena.

## Claims

1. An intraocular lens, IOL, (100), comprising:
a lens body (102) having a monolithic anterior lens element (102A) having an anterior nanostructure assembly (110A) formed thereon and a monolithic posterior lens element (102P) having a posterior nanostructure assembly (110P) formed thereon;
an optical fluid filled in a cavity (106) formed between the monolithic anterior lens element (102A) and the monolithic posterior lens element (102P); and
one or more haptics (104) coupled to the lens body (102).

2. The IOL of claim 1, wherein the monolithic anterior lens element (102A) and the monolithic posterior lens element (102P) each comprises poly (dimethylsiloxane), PDMS.

3. The IOL of claim 1, wherein the optical fluid comprises silicone oil.

4. The IOL of claim 1, wherein the one or more haptics (104) each include an internal volume that is in fluid communication with the cavity (106).

5. The IOL of claim 1, wherein
each of the anterior nanostructure assembly (110A) and the posterior nanostructure assembly (110P) includes a plurality of protrusions (112) having heights of between 30 nm and 200 nm, widths of between 30 nm and 300 nm, and spacings between adjacent protrusions of between 30 nm and 300 nm.

6. The IOL of claim 1, wherein
the anterior nanostructure assembly and the posterior nanostructure assembly are configured to reduce reflectivity of the lens body by between 10 % and 90 % as compared to a lens body with no nanostructure assemblies (110A, 110P) on the monolithic anterior lens element (102A) or the monolithic posterior lens element (102P).

7. A method for fabricating an intraocular lens, IOL (100), comprising:
fabricating a monolithic anterior lens element (102A) and a monolithic posterior lens element (102P), wherein
the monolithic anterior lens element (102A) has an anterior nanostructure assembly (110A) formed thereon, and
the monolithic posterior lens element (102P) has a posterior nanostructure assembly (110P) formed thereon;
bonding the monolithic anterior lens element (102A) and the monolithic posterior lens element (102P) to form a cavity (106) therebetweeen; and
filling the cavity (106) with an optical fluid.

8. The method of claim 7, wherein
the fabricating of each of the monolithic anterior lens element (102A) and the monolithic posterior lens element (102P) comprises:
casting (320) an elastomeric membrane (408) on a substrate (404) having a nanostructured pattern (402) formed thereon; and
removing the elastomeric membrane (408) from the substrate (404).

9. The method of claim 8, wherein
the elastomeric membrane (408) comprises poly (dimethylsiloxane) (PDMS).

10. The method of claim 8, wherein
the nanostructured pattern (402) formed on the substrate (404) extends over an area of 7 mm and 7 mm, and includes an array of trenches (406) with heights of between 30 nm and 200 nm, widths of between 30 nm and 300 nm, and spacings between adjacent trenches (406) of between 30 nm and 300 nm.

11. The method of claim 7, wherein
the anterior nanostructure assembly (110A) and the posterior nanostructure assembly (110P) each include a plurality of protrusions (112) having heights of between 30 nm and 200 nm, widths of between 30 nm and 300 nm, and spacings between adjacent protrusions (112) of between 30 nm and 300 nm.

12. The method of claim 7, wherein
the optical fluid comprises silicone oil.

13. The method of claim 7, further comprising:
attaching a haptic portion (104) to a peripheral non-optic portion of the monolithic anterior lens element and the monolithic posterior lens element (102P),
wherein the haptic portion (104) comprises one or more hollow haptics (104A, 104B) having an internal volume that is in fluid communication with the cavity (106).

## Patentansprüche

1. Intraokularlinse, IOL, (100), umfassend:
einen Linsenkörper (102) mit einem monolithischen anterioren Linsenelement (102A) mit einer darauf ausgebildeten anterioren Nanostrukturbaugruppe (110A) und einem monolithischen posterioren Linsenelement (102P) mit einer darauf ausgebildeten posterioren Nanostrukturbaugruppe (110P);
ein optisches Fluid, das in einen Hohlraum (106) gefüllt ist, der zwischen dem monolithischen anterioren Linsenelement (102A) und dem monolithischen posterioren Linsenelement (102P) ausgebildet ist; und
eine oder mehrere Haptiken (104), die mit dem Linsenkörper (102) gekoppelt sind.

2. IOL nach Anspruch 1, wobei das monolithische anteriore Linsenelement (102A) und das monolithische posteriore Linsenelement (102P) jeweils Poly(dimethylsiloxan), PDMS, enthalten.

3. IOL nach Anspruch 1, wobei das optische Fluid Silikonöl enthält.

4. IOL nach Anspruch 1, wobei die eine oder die mehreren Haptiken (104) jeweils ein Innenvolumen aufweisen, das in Fluidverbindung mit dem Hohlraum (106) steht.

5. IOL nach Anspruch 1, wobei
die anteriore Nanostrukturbaugruppe (110A) und die posteriore Nanostrukturbaugruppe (110P) jeweils mehrere Vorsprünge (112) mit Höhen zwischen 30 nm und 200 nm, Breiten zwischen 30 nm und 300 nm und Abständen zwischen benachbarten Vorsprüngen zwischen 30 nm und 300 nm aufweisen.

6. IOL nach Anspruch 1, wobei
die anteriore Nanostrukturbaugruppe und die posteriore Nanostrukturbaugruppe dazu ausgelegt sind, einen Reflexionsgrad des Linsenkörpers um einen Betrag zwischen 10 % und 90 % im Vergleich zu einem Linsenkörper ohne Nanostrukturbaugruppen (110A, 110P) auf dem monolithischen anterioren Linsenelement (102A) oder dem monolithischen posterioren Linsenelement (102P) zu reduzieren.

7. Verfahren zum Herstellen einer Intraokularlinse, IOL (100), umfassend:
Herstellen eines monolithischen anterioren Linsenelements (102A) und eines monolithischen posterioren Linsenelements (102P), wobei das monolithische anteriore Linsenelement (102A) eine darauf ausgebildete anteriore Nanostrukturbaugruppe (110A) aufweist, und
das monolithische posteriore Linsenelement (102P) eine darauf ausgebildete posteriore Nanostrukturbaugruppe (110P) aufweist;
Bonden des monolithischen anterioren Linsenelements (102A) und des monolithischen posterioren Linsenelements (102P), um dazwischen einen Hohlraum (106) auszubilden; und
Füllen des Hohlraums (106) mit einem optischen Fluid.

8. Verfahren nach Anspruch 7, wobei
das Herstellen jeweils des monolithischen anterioren Linsenelements (102A) und des monolithischen posterioren Linsenelements (102P) umfasst:
Gießen (320) einer elastomeren Membran (408) auf ein Substrat (404) mit einem darauf ausgebildeten nanostrukturierten Muster (402); und
Entfernen der elastomeren Membran (408) von dem Substrat (404).

9. Verfahren nach Anspruch 8, wobei
die elastomere Membran (408) Poly(dimethylsiloxan) (PDMS) enthält.

10. Verfahren nach Anspruch 8, wobei
das auf dem Substrat (404) ausgebildete nanostrukturierte Muster (402) sich über eine Fläche von 7 mm mal 7 mm erstreckt und ein Array von Gräben (406) mit Höhen zwischen 30 nm und 200 nm, Breiten zwischen 30 nm und 300 nm und Abständen zwischen benachbarten Gräben (406) zwischen 30 nm und 300 nm aufweist.

11. Verfahren nach Anspruch 7, wobei
die anteriore Nanostrukturbaugruppe (110A) und die posteriore Nanostrukturbaugruppe (110P) jeweils mehrere Vorsprünge (112) mit Höhen zwischen 30 nm und 200 nm, Breiten zwischen 30 nm und 300 nm und Abständen zwischen benachbarten Vorsprüngen (112) zwischen 30 nm und 300 nm aufweisen.

12. Verfahren nach Anspruch 7, wobei
das optische Fluid Silikonöl enthält.

13. Verfahren nach Anspruch 7, ferner umfassend:
Anbringen eines haptischen Abschnitts (104) an einem peripheren nichtoptischen Abschnitt des monolithischen anterioren Linsenelements und des monolithischen posterioren Linsenelements (102P),
wobei der haptische Abschnitt (104) eine oder mehrere hohle Haptiken (104A, 104B) mit einem Innenvolumen, das in Fluidverbindung mit dem Hohlraum (106) steht, aufweist.

## Revendications

1. Lentille intraoculaire, IOL, (100) comprenant :
un corps de lentille (102) comprenant un élément de lentille antérieur monolithique (102A) sur lequel est formé un ensemble de nanostructures antérieur (110A) et un élément de lentille postérieur monolithique (102P) sur lequel est formé un ensemble de nanostructures postérieur (110P) ;
un fluide optique contenu dans une cavité (106) formée entre l'élément de lentille antérieur monolithique (102A) et l'élément de lentille postérieur monolithique (102P) ; et
un ou plusieurs haptiques (104) couplés au corps de lentille (102).

2. IOL selon la revendication 1, dans laquelle l'élément de lentille antérieur monolithique (102A) et l'élément de lentille postérieur monolithique (102P) comprennent chacun du poly(diméthylsiloxane), PDMS.

3. IOL selon la revendication 1, dans laquelle le fluide optique comprend de l'huile de silicone.

4. IOL selon la revendication 1, dans laquelle les un ou plusieurs haptiques (104) comprennent chacun un volume interne qui est en communication fluidique avec la cavité (106).

5. IOL selon la revendication 1, dans laquelle :
chacun de l'ensemble de nanostructures antérieur (110A) et de l'ensemble de nanostructures postérieur (110P) comprend une pluralité de saillies (112) ayant des hauteurs comprises entre 30 nm et 200 nm, des largeurs comprises entre 30 nm et 300 nm, et des espacements entre des saillies adjacentes compris entre 30 nm et 300 nm.

6. IOL selon la revendication 1, dans laquelle :
l'ensemble de nanostructures antérieur et l'ensemble de nanostructures postérieur sont configurés pour réduire la réflectivité du corps de lentille d'entre 10 % et 90 % par rapport à un corps de lentille ne comportant aucun ensemble de nanostructures (110A, 110P) sur l'élément de lentille antérieur monolithique (102A) ou l'élément de lentille postérieur monolithique (102P).

7. Procédé de fabrication d'une lentille intraoculaire, IOL (100), comprenant les étapes suivantes :
fabriquer un élément de lentille antérieur monolithique (102A) et un élément de lentille postérieur monolithique (102P), où
l'élément de lentille antérieur monolithique (102A) comporte un ensemble de nanostructures antérieur (110A) formé sur celui-ci, et
l'élément de lentille postérieur monolithique (102P) comporte un ensemble de nanostructures postérieur (110P) formé sur celui-ci ;
coller l'élément de lentille antérieur monolithique (102A) et l'élément de lentille postérieur monolithique (102P) pour former une cavité (106) entre eux ; et
remplir la cavité (106) avec un fluide optique.

8. Procédé selon la revendication 7, dans lequel :
la fabrication de chacun parmi l'élément de lentille antérieur monolithique (102A) et l'élément de lentille postérieur monolithique (102P) comprend les étapes suivantes :
couler (320) une membrane élastomère (408) sur un substrat (404) sur lequel est formé un motif nanostructuré (402) ; et
enlever la membrane élastomère (408) du substrat (404).

9. Procédé selon la revendication 8, dans lequel :
la membrane élastomère (408) comprend du poly(diméthylsiloxane), PDMS.

10. Procédé selon la revendication 8, dans lequel :
le motif nanostructuré (402) formé sur le substrat (404) s'étend sur une surface de 7 mm par 7 mm, et comporte un réseau de tranchées (406) ayant des hauteurs comprises entre 30 nm et 200 nm, des largeurs comprises entre 30 nm et 300 nm, et des espacements entre tranchées adjacentes (406) compris entre 30 nm et 300 nm.

11. Procédé selon la revendication 7, dans lequel :
l'ensemble de nanostructures antérieur (110A) et l'ensemble de nanostructures postérieur (110P) comprennent chacun une pluralité de saillies (112) ayant des hauteurs comprises entre 30 nm et 200 nm, des largeurs comprises entre 30 nm et 300 nm, et des espacements entre des saillies (112) adjacentes compris entre 30 nm et 300 nm.

12. Procédé selon la revendication 7, dans lequel :
le fluide optique comprend de l'huile de silicone.

13. Procédé selon la revendication 7, comprenant en outre les étapes suivantes :
fixer une partie haptique (104) à une partie non optique périphérique de l'élément de lentille antérieure monolithique et de l'élément de lentille postérieure monolithique (102P),
où la partie haptique (104) comprend une ou plusieurs parties haptiques creuses (104A, 104B) ayant un volume interne qui est en communication fluidique avec la cavité (106).
